# EUROPEAN PATENT APPLICATION

(11) **EP 4 789 616 A1**
(43) Date of publication of application: **12.08.2026**
(21) Application number: 26156463.7
(22) Date of filing: 04.02.2026
(51) Int. Cl.: A61B 6/06, A61B 6/51, A61B 6/00, A61B 6/50

(54) **MULTI APERTURE PANORAMIC X-RAY IMAGING**

(30) Priority: 05.02.2025 US 202519046263
(71) Applicant: Zetta25 AG, 8047 Zurich (CH)
(72) Inventor: EICHNER, Stefan, 69123 Heidelberg (DE)
(74) Representative: Taor, Simon Edward William

(57) **Abstract**

Techniques and apparatuses are described that perform panoramic X-ray imaging. In an example aspect, the techniques and apparatuses perform projecting a main X-ray beam onto a collimator to form a plurality of individual X-ray projection beams, where the collimator includes a plurality of apertures to convert the main X-ray beam into the plurality of individual X-ray projection beams, imaging a dental area of a patient by projecting the plurality of individual X-ray projection beams through the dental area, recording a plurality of X-ray projection images corresponding to an each individual X-ray projection beam of the plurality of individual X-ray projection beams onto a detector, based on the desired scanning trajectory, and reconstructing a panoramic X-ray image of the dental area based on the plurality of X-ray projection images corresponding to the each individual X-ray projection beam of the plurality of individual X-ray projection beams.

## Description

### FIELD

The present disclosure generally relates to panoramic X-ray imaging and more particularly to a panoramic X-ray imaging of a dental area using multiple X-ray projection beams.

### BACKGROUND

Radiography images, such as X-rays, are commonly used as a standard procedure for diagnosing various medical and dental conditions. Different types of imaging devices are used for diagnosis, including extraoral X-ray imaging devices that capture panoramic radiography or X-ray images of a body part of a patient. For diagnosis of dental conditions, the panoramic X-ray images provide a comprehensive view of the jaws of the patient including the upper and the lower jaws, the temporomandibular joint, and surrounding structures. In practice, an extraoral X-ray imaging device acquires multiple two-dimensional (2D) X-ray images at different angles by moving along a trajectory around the body part such as head of the patient. These 2D X-ray images are then combined to reconstruct a final panoramic X-ray image.

### SUMMARY

According to an embodiment of the present disclosure, a method is disclosed that includes projecting a main X-ray beam onto a collimator to form a plurality of individual X-ray projection beams. The collimator includes a plurality of apertures to convert the main X-ray beam into the plurality of individual X-ray projection beams. The method further includes imaging a dental area of a patient by projecting the plurality of individual X-ray projection beams through the dental area based on a desired scanning trajectory. The method further includes recording a plurality of X-ray projection images corresponding to each individual X-ray projection beam of the plurality of individual X-ray projection beams using a detector. The method also includes reconstructing a panoramic X-ray image of the dental area based on one or more of the plurality of X-ray projection images corresponding to the individual X-ray projection beams.

In an embodiment, the method may also include reconstructing the panoramic X-ray image based on the one of the plurality of X-ray projection images. In another embodiment, the method may also include reconstructing the panoramic X-ray image based on a combination of the plurality of X-ray projection images. In another embodiment, the method may also include reconstructing the panoramic X-ray image based on all X-ray projection images corresponding to the plurality of individual X-ray projection beams.

In an embodiment, the method may also include reconstructing the X-ray image of the dental area based on at least a sequential or a parallel read-out of the plurality of X-ray projection images.

Aspects described below include a non-transitory computer-readable storage medium comprising computer-executable instructions that, responsive to execution by a processor, cause a system to perform any one of the described methods.

Aspects described below also include a system with means for performing panoramic X-ray imaging.

### BRIEF DESCRIPTION OF THE DRAWINGS

Apparatuses for and techniques for panoramic X-ray imaging are described with reference to the following drawings. The same numbers are used throughout the drawings to reference like features and components. The drawings are of illustrative embodiments. They do not illustrate all embodiments. Other embodiments may be used in addition or instead. Details that may be apparent or unnecessary may be omitted to save space or for more effective illustration. Some embodiments may be practiced with additional components or steps and/or without all the components or steps that are illustrated. When the same numeral appears in different drawings, it refers to the same or like components or steps.
FIG. 1 depicts a block diagram of a data processing environment in which illustrative embodiments may be implemented.
FIG. 2 depicts a block diagram of a data processing system in which illustrative embodiments may be implemented.
FIG. 3A depicts a view of a conventional panoramic X-ray device in accordance with a prior art.
Fig. 3B depicts a view of a collimator of the conventional panoramic X-ray device as shown in FIG. 3A in accordance with the prior art.
FIG. 4 depicts a view of a panoramic X-ray device in accordance with an illustrative embodiment.
FIG. 5A depicts a view of a collimator of a panoramic X-ray device in accordance with an illustrative embodiment.
FIG. 5B depicts a view of a collimator of a panoramic X-ray device in accordance with an illustrative embodiment.
FIG. 6A depicts a view of a detector of a panoramic X-ray device corresponding to the collimator as shown in FIG. 5A in accordance with an illustrative embodiment.
FIG. 6B depicts a view of a detector of a panoramic X-ray device corresponding to the collimator as shown in FIG. 5B in accordance with an illustrative embodiment.
FIG. 7A depicts an X-ray image of the dental area reconstructed using X-ray projection images corresponding to one individual X-ray projection beam in accordance with an illustrative embodiment.
FIG. 7B depicts an X-ray image of the dental area reconstructed using X-ray projection images corresponding to another individual X-ray projection beam in accordance with an illustrative embodiment.
FIG. 7C depicts an X-ray image of the dental area reconstructed based on a default focal layer position, in accordance with an illustrative embodiment.
Fig. 7D depicts X-ray images of the dental area reconstructed based on alternative focal layer positions using X-ray projection images corresponding to a plurality of individual X-ray projection beams in accordance with an illustrative embodiment.
FIG. 8 depicts a method for panoramic X-ray imaging in accordance with an illustrative embodiment.

### DETAILED DESCRIPTION

### Overview

In the following detailed description, numerous specific details are set forth by way of examples to provide a thorough understanding of the relevant teachings. However, it should be apparent that the present teachings may be practiced without such details. In other instances, well-known methods, procedures, components, and/or circuitry have been described at a relatively high level, without detail, to avoid unnecessarily obscuring aspects of the present teachings.

Panoramic X-ray imaging is a widely used dental imaging technique that provides a comprehensive view of the upper and lower jaw, including the temporomandibular joints of a patient. Generally, in panoramic X-ray imaging, the panoramic X-ray image is generated by rotating an X-ray source and a detector around a patient's head along a desired scanning trajectory. Simultaneously, an X-ray beam is projected from the X-ray source onto the detector through a dental part to record the X-ray projection images. The X-ray projection images are then used to reconstruct a panoramic X-ray image of the dental part. Further, the X-ray beam from the X-ray source is projected onto the dental part through a collimator which controls the X-ray radiation only to the dental part that is to be imaged.

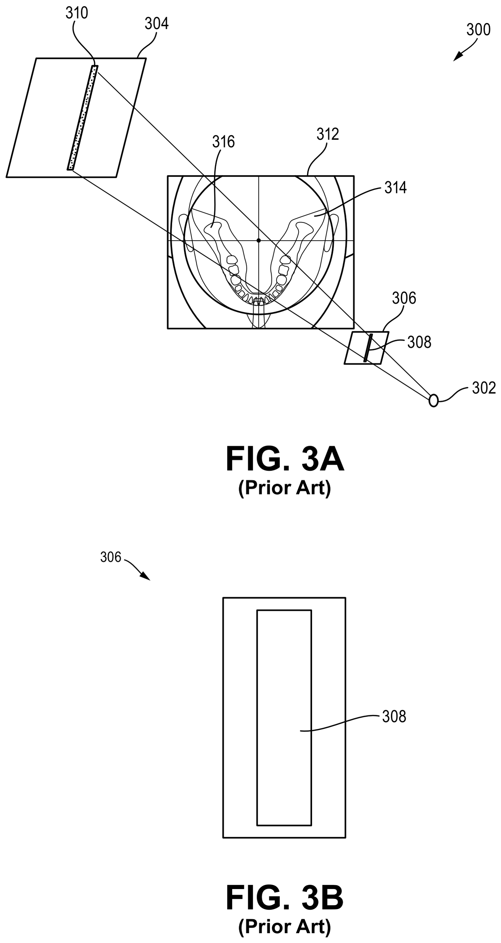

The illustrative embodiments recognize that a conventional panoramic X-ray imaging device 300, as shown in FIG. 3A may include an X-ray source 302, a recorder/detector 304, and a collimator 306. The X-ray source 302 and the recorder 304 are configured across the patient's head 312 and rotate reciprocally along the predefined scanning trajectory 316 to scan specific dental part 314. Further, the collimator 306 with an aperture 308 is configured between the X-ray source 302 and the patient's head 312. The X-ray beam is projected onto the dental part 314 of the patient's head 312 through the aperture 308. The X-ray beam is projected along the predefined scanning trajectory to record a plurality of X-ray projection images 310 on the recorder 304. Further, the plurality of X-ray projection images 310 is used to generate the panoramic X-ray image of the dental part 314.

Traditionally, the collimator 306 includes a single aperture 308, as shown in FIG. 3B (prior art). The aperture 308 is size adjustable to control the amount and angle of projection of X-ray radiation depending on a type of scanning program such as an upper jaw scanning, a lower jaw scanning, or a complete dental structure. However, the size of the aperture 308 has a significant impact on the reconstructed panoramic X-ray image. A wider aperture 308 reduces the ability to detect individual dental structures and also the possibility of optimizing the panoramic X-ray image using conventional autofocus methods. This makes diagnosis more difficult in the affected areas of the panoramic X-ray image. Further, the wider aperture 308 increases the amount of radiation on the patient's head 312. On the other hand, a thinner aperture 308 reduces the angle of projection for each individual dental structure limiting data for reconstruction of the panoramic X-ray image. This leads to unfavorable overlapping of the individual dental structures (teeth) within the panoramic X-ray image, which in turn makes diagnosis more difficult in the affected regions of the panoramic image, as overlapping proximal contacts (tooth crowns) often occur due to the beam angles.

Further, the traditional panoramic X-ray imaging device 300 uses a dedicated panoramic detector with a narrow sensor width and correspondingly narrow aperture 308 of the collimator 306 or a flat panel detector with a narrow readout region (partial readout) at a fixed, predefined sensor position and a correspondingly narrow aperture 308 of the collimator 306 for panoramic X-ray imaging. The aperture 308 is located between the patient's head 312 and the X-ray source 302 and limits the applied radiation depending on the type of panoramic image selected.

Techniques described herein implement panoramic X-ray imaging utilizing a multi-aperture collimator and subsequent reconstruction of x-ray images. In example aspects, a method is disclosed that includes projecting a main X-ray beam onto a collimator to form a plurality of individual X-ray projection beams. The collimator includes a plurality of apertures to convert the main X-ray beam into the plurality of individual X-ray projection beams. The method further includes imaging a dental area of a patient based on the plurality of individual X-ray projection beams being directed through the dental area. The method further includes recording a plurality of X-ray projection images corresponding to each individual X-ray projection beam of the plurality of individual X-ray projection beams on a detector. The method also includes reconstructing a panoramic X-ray image of the dental area based on the plurality of X-ray projection images corresponding to the individual X-ray projection beams.

The illustrative embodiments are described with respect to certain types of machines. The illustrative embodiments are also described with respect to other scenes, subjects, measurements, devices, data processing systems, environments, components, and applications only as examples. Any specific manifestations of these and other similar artifacts are not intended to be limiting to the disclosure. Any suitable manifestation of these and other similar artifacts can be selected within the scope of the illustrative embodiments.

Furthermore, the illustrative embodiments may be implemented with respect to any type of data, data source, or access to a data source over a data network. Any type of data storage device may provide the data to an embodiment of the disclosure, either locally at a data processing system or over a data network, within the scope of the disclosure. Where an embodiment is described using a mobile device, any type of data storage device suitable for use with the mobile device may provide the data to such embodiment, either locally at the mobile device or over a data network, within the scope of the illustrative embodiments.

The illustrative embodiments are described using specific surveys, code, hardware, algorithms, designs, architectures, protocols, layouts, schematics, and tools only as examples and are not limiting to the illustrative embodiments. Furthermore, the illustrative embodiments are described in some instances using particular software, tools, and data processing environments only as an example for the clarity of the description. The illustrative embodiments may be used in conjunction with other comparable or similarly purposed structures, systems, applications, or architectures. For example, other comparable devices, structures, systems, applications, or architectures therefor, may be used in conjunction with such embodiment of the disclosure within the scope of the disclosure. An illustrative embodiment may be implemented in hardware, software, or a combination thereof.

The examples in this disclosure are used only for the clarity of the description and are not limiting to the illustrative embodiments. Additional data, operations, actions, tasks, activities, and manipulations will be conceivable from this disclosure and the same are contemplated within the scope of the illustrative embodiments.

Any advantages listed herein are only examples and are not intended to be limiting to the illustrative embodiments. Additional or different advantages may be realized by specific illustrative embodiments. Furthermore, a particular illustrative embodiment may have some, all, or none of the advantages listed above.

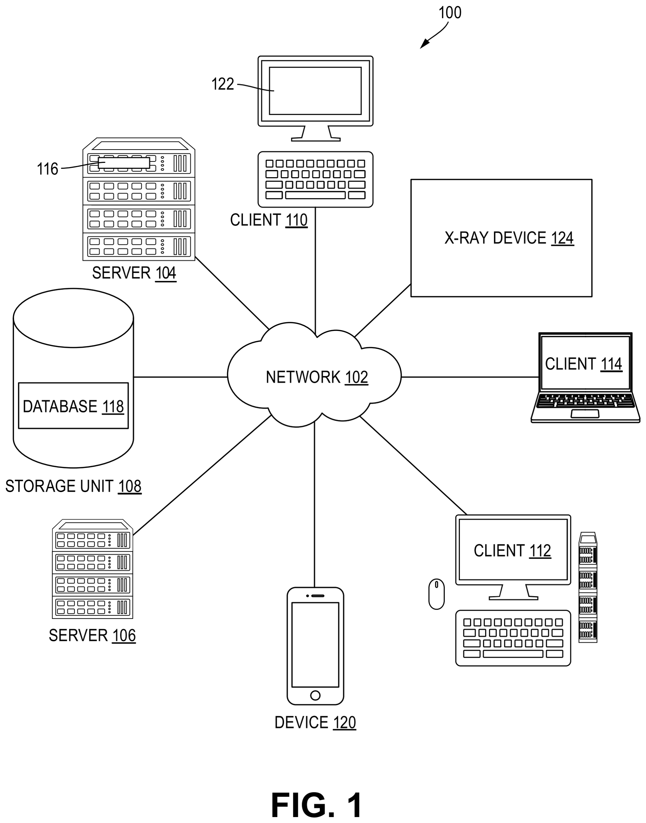

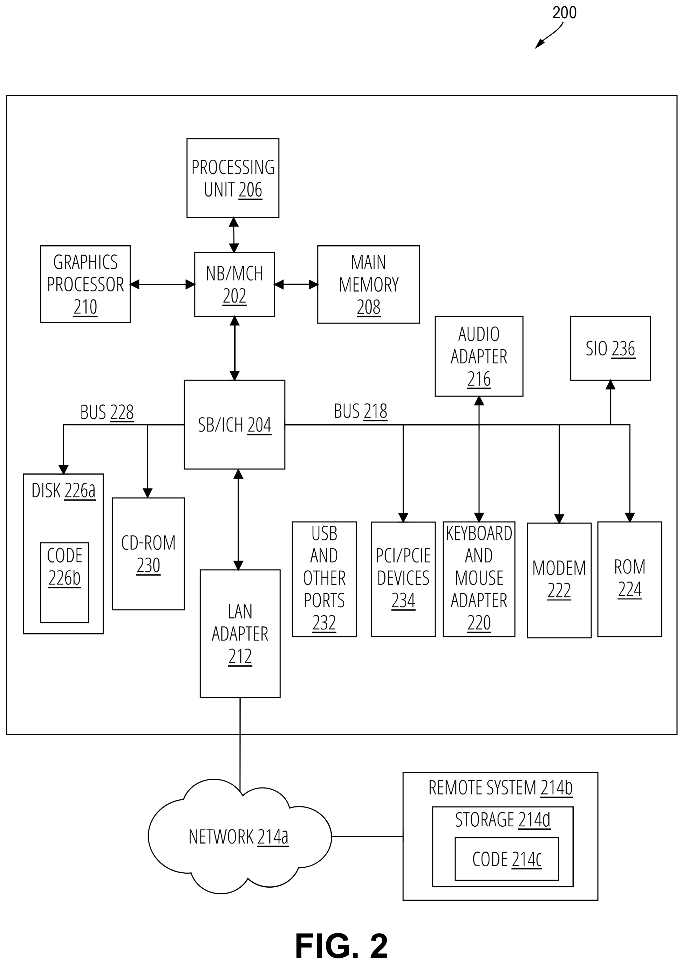

With reference to the figures and in particular, with reference to FIG. 1 and FIG. 2, these figures are example diagrams of data processing environments in which illustrative embodiments may be implemented. FIG. 1 and FIG. 2 are only examples and are not intended to assert or imply any limitation with regard to the environments in which different embodiments may be implemented. A particular implementation may make many modifications to the depicted environments based on the following description.

FIG. 1 depicts a block diagram of a network of data processing systems in which illustrative embodiments may be implemented. Data processing environment 100 is a network of computers in which the illustrative embodiments may be implemented. Data processing environment 100 includes network 102. Network 102 is the medium used to provide communications links between various devices and computers connected together within the data processing environment 100. Network 102 may include connections, such as wired, wireless communication links, or fiber optic cables.

Clients or servers are only example roles of certain data processing systems connected to network 102 and are not intended to exclude other configurations or roles for these data processing systems. Server 104 and server 106 couple to network 102 along with storage unit 108. Software applications may execute on any computer in data processing environment 100. Client 110, client 112, and client 114 are also coupled to network 102. A data processing system, such as server 104 or server 106, or clients (client 110, client 112, client 114) may contain data and may have software applications or software tools executing thereon. Server 104 may include one or more GPUs (graphics processing units) for training one or more models.

Only as an example, and without implying any limitation to such architecture, FIG. 1 depicts certain components that are usable in an example implementation of an embodiment. For example, servers and clients are only examples and not to imply a limitation to a client-server architecture. As another example, an embodiment can be distributed across several data processing systems and a data network as shown, whereas another embodiment can be implemented on a single data processing system within the scope of the illustrative embodiments. Data processing systems (server 104, server 106, client 110, client 112, client 114) also represent example nodes in a cluster, partitions, and other configurations suitable for implementing an embodiment.

Device 120 is an example of a device described herein. For example, device 120 can take the form of a smartphone, a special purpose fabrication platform, a tablet computer, a laptop computer, client 110 in a stationary or a portable form, a wearable computing device, or any other suitable device. Any software application described as executing in another data processing system in FIG. 1 can be configured to execute in device 120 in a similar manner. Any data or information stored or produced in another data processing system in FIG. 1 can be configured to be stored or produced in device 120 in a similar manner.

X-ray device 124 may execute as a device that includes the client application 122, server application 116, or any data processing system herein. X-ray device 124 may also execute as a cloud service communicatively coupled to system services, hardware resources, or software elements described herein. Database 118 of the storage unit 108 stores one or more data in repositories for computations herein. X-ray device 124 may perform the method comprising projecting a main X-ray beam onto a collimator to form a plurality of individual X-ray projection beams. A collimator of the x-ray device 124 includes a plurality of apertures to convert the main X-ray beam into the plurality of individual X-ray projection beams.

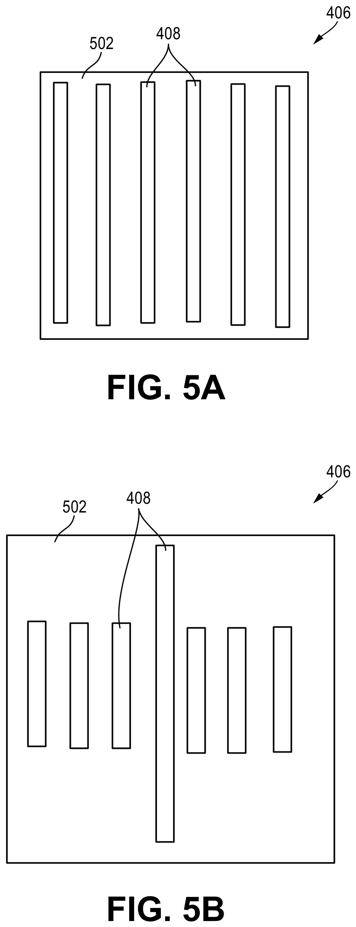

The use of multiple apertures can improve the diagnostic possibilities and the image quality of panoramic images on extraoral X-ray devices without additional / increased dose exposure for the patient and with identical diagnostic regions for all conventional panoramic program types. This is achieved by using the special multi-slit aperture in combination with a flat-panel sensor and corresponding data readout and subsequent reconstruction. The trajectories of the panorama program types can remain unchanged. Special artifact-reduced panorama program types can be omitted. The special multi-slit diaphragm/collimator enables a higher angular coverage during panoramic exposures with identical or lower dose exposure for the patient. This can be used alone or in combination with a motorized diaphragm (additional collimation) on the radiator side. Due to the higher angular coverage in the rotation of the panoramic images, more dental structures of the mandibular arch can be captured orthoradially during the rotation. The higher angle coverage enables additional reconstruction options for panoramic images with a significant reduction in overlapping dental crowns, which in turn improves the diagnostic options. This may help with diagnosis, especially for caries detection. Higher angular coverage results in thinner layers in the standard reconstruction, which in turn improves the autofocus options, especially in the molar region, and increases the overall image quality of the panoramic images. The multiple slit apertures can be designed differently locally (e.g., greater angular coverage in the area of the mandibular arch, standard position for the eye socket and the lower chin area, see FIGS. 5 - 6). The parallel or sequential readout of the flat panel sensor enables the areas of the sensor defined by the multiple slit apertures to be captured locally. The frame rate can be similar to a classic panoramic image, as the sum of the area of the areas does not have to be greater than in a classic panoramic image. Since the geometry of the resulting projection images is known, the software reconstruction can calculate different panoramic images (standard view, partial reconstructions) depending on the diagnostic requirement as well as an autofocus-optimized panoramic image.

The readout areas of the flat panel sensor can be selected / oriented in such a way that the irradiated areas of the detector/sensor defined by the special multi-slot aperture are detected. The readout rate (parallel or sequential) of the flat panel sensor can be selected to be as high as possible. A total readout of the flat panel sensor is therefore not necessarily possible. The individual images per readout area of the flat panel sensor for each readout time point are recorded and made available for reconstruction. Knowing the recording geometry of all individual images, this initially reconstructs the panoramic image based on the focal curve resulting from the path curve (standard layer). Possible additional reconstruction variants to improve the diagnostic possibilities and image quality can be performed. These include autofocus optimized standard slice of the panoramic image over the entire mandibular arch, additional (local) slice layers within the entire mandibular arch position determination of objects (e.g., for implant planning), and additional (local) angular views opening of overlapping proximal contacts within the mandibular arch (e.g., for caries detection).

Server application 116 implements an embodiment described herein. Server application 116 can use data from storage unit 108 for panoramic X-ray imaging. Server application 116 can also obtain data from any client for computations. Server application 116 can also execute in any of data processing systems (server 104 or server 106, client 110, client 112, client 114), such as client application 122 in client 110, and need not execute in the same system as server 104.

Server 104, server 106, storage unit 108, client 110, client 112, client 114, and device 120 may couple to network 102 using wired connections, wireless communication protocols, or other suitable data connectivity. Client 110, client 112, and client 114 may be, for example, personal computers or network computers.

In the depicted example, server 104 may provide data, such as boot files, operating system images, and applications to client 110, client 112, and client 114. Client 110, client 112, and client 114 may be clients to server 104 in this example. Client 110, client 112, and client 114 or some combination thereof, may include their own data, boot files, operating system images, and applications. Data processing environment 100 may include additional servers, clients, and other devices that are not shown. Server 104 includes a server application 116 that may be configured to implement one or more of the functions described herein in accordance with one or more embodiments.

The data processing environment 100 may also be the Internet. Network 102 may represent a collection of networks and gateways that use the Transmission Control Protocol/Internet Protocol (TCP/IP) and other protocols to communicate with one another. At the heart of the Internet is a backbone of data communication links between major nodes or host computers, including thousands of commercial, governmental, educational, and other computer systems that route data and messages. Of course, data processing environment 100 also may be implemented as a number of different types of networks, such as for example, an intranet, a local area network (LAN), or a wide area network (WAN). FIG. 1 is intended as an example, and not as an architectural limitation for the different illustrative embodiments.

Among other uses, data processing environment 100 may be used for implementing a client-server environment in which the illustrative embodiments may be implemented. A client-server environment enables software applications and data to be distributed across a network such that an application functions by using the interactivity between a client data processing system and a server data processing system. Data processing environment 100 may also employ a service-oriented architecture where interoperable software components distributed across a network may be packaged together as coherent business applications. Data processing environment 100 may also take the form of a cloud and employ a cloud computing model of service delivery for enabling convenient, on-demand network access to a shared pool of configurable computing resources (e.g., networks, network bandwidth, servers, processing, memory, storage, applications, virtual machines, and services) that can be rapidly provisioned and released with minimal management effort or interaction with a provider of the service.

With reference to FIG. 2, this figure depicts a block diagram of a data processing system in which illustrative embodiments may be implemented. Data processing system 200 is an example of a computer, such as server 104, server 106, or client 110, client 112, client 114, X-ray device 124 in FIG. 1, or another type of device in which computer-usable program code or instructions implementing the processes may be located for the illustrative embodiments.

Data processing system 200 is also representative of a data processing system or a configuration therein, such as device 120 in FIG. 1 in which computer-usable program code or instructions implementing the processes of the illustrative embodiments may be located. Data processing system 200 is described as a computer only as an example, without being limited thereto. Implementations in the form of other devices, such as device 120 in FIG. 1, may modify data processing system 200, such as by adding a touch interface, and even eliminate certain depicted components from data processing system 200 without departing from the general description of the operations and functions of data processing system 200 described herein.

In the depicted example, data processing system 200 employs a hub architecture including North Bridge and memory controller hub (NB/MCH) 202 and South Bridge and input/output (I/O) controller hub (SB/ICH) 204. Processing unit 206, main memory 208, and graphics processor 210 are coupled to North Bridge and memory controller hub (NB/MCH) 202. Processing unit 206 may contain one or more processors and may be implemented using one or more heterogeneous processor systems. Processing unit 206 may be a multi-core processor. Graphics processor 210 may be coupled to North Bridge and memory controller hub (NB/MCH) 202 through an accelerated graphics port (AGP) in certain implementations.

In the depicted example, local area network (LAN) adapter 212 is coupled to South Bridge and input/output (I/O) controller hub (SB/ICH) 204. Audio adapter 216, keyboard and mouse adapter 220, modem 222, read only memory (ROM) 224, universal serial bus (USB) and other ports 232, and PCI/PCIe devices 234 are coupled to South Bridge and input/output (I/O) controller hub (SB/ICH) 204 through bus 218. Hard disk drive (HDD) or solid-state drive (SSD) 226a and CD-ROM 230 are coupled to South Bridge and input/output (I/O) controller hub (SB/ICH) 204 through bus 228. PCI/PCIe devices 234 may include, for example, Ethernet adapters, add-in cards, and PC cards for notebook computers. PCI uses a card bus controller, while PCIe does not. Read only memory (ROM) 224 may be, for example, a flash binary input/output system (BIOS). Hard disk drive (HDD) or solid-state drive (SSD) 226a and CD-ROM 230 may use, for example, an integrated drive electronics (IDE), serial advanced technology attachment (SATA) interface, or variants such as external-SATA (eSATA) and micro- SATA (mSATA). A super I/O (SIO) device 236 may be coupled to South Bridge and input/output (I/O) controller hub (SB/ICH) 204 through bus 218.

Memories, such as main memory 208, read-only memory (ROM) 224, or flash memory (not shown), are some examples of computer usable storage devices. Hard disk drive (HDD) or solid-state drive (SSD) 226a, CD-ROM 230, and other similarly usable devices are some examples of computer usable storage devices including a computer usable storage medium.

An operating system runs on processing unit 206. The operating system coordinates and provides control of various components within data processing system 200 in FIG. 2. The operating system may be a commercially available operating system for any type of computing platform, including but not limited to server systems, personal computers, and mobile devices. An object-oriented or other type of programming system may operate in conjunction with the operating system and provide calls to the operating system from programs or applications executing on data processing system 200.

Instructions for the operating system, the object-oriented programming system, and applications or programs, such as server application 116 and client application 122 in FIG. 1, are located on storage devices, such as in the form of codes 226b on Hard disk drive (HDD) or solid-state drive (SSD) 226a, and may be loaded into at least one of one or more memories, such as main memory 208, for execution by processing unit 206. The processes of the illustrative embodiments may be performed by processing unit 206 using computer-implemented instructions, which may be located in a memory, such as, for example, main memory 208, read-only memory (ROM) 224, or in one or more peripheral devices.

Furthermore, in one case, code 226b may be downloaded over network 214a from remote system 214b, where similar code 214c is stored on a storage device 214d in another case, code 226b may be downloaded over network 214a to remote system 214b, where downloaded code 214c is stored on a storage device 214d.

The hardware in FIG. 1 and FIG. 2 may vary depending on the implementation. Other internal hardware or peripheral devices, such as flash memory, equivalent non-volatile memory, or optical disk drives and the like, may be used in addition to or in place of the hardware depicted in FIG. 1 and FIG. 2. In addition, the processes of the illustrative embodiments may be applied to a multiprocessor data processing system.

In some illustrative examples, data processing system 200 may be a personal digital assistant (PDA), which is generally configured with flash memory to provide non-volatile memory for storing operating system files and/or user-generated data. A bus system may comprise one or more buses, such as a system bus, an I/O bus, and a PCI bus. Of course, the bus system may be implemented using any type of communications fabric or architecture that provides for a transfer of data between different components or devices attached to the fabric or architecture.

A communications unit may include one or more devices used to transmit and receive data, such as a modem or a network adapter. A memory may be, for example, a main memory 208 or a cache, such as the cache found in North Bridge and memory controller hub (NB/MCH) 202. A processing unit may include one or more processors or CPUs.

The depicted examples in FIG. 1 and FIG. 2 and the above-described examples are not meant to imply architectural limitations. For example, Data Processing System 200 also may be a tablet computer, laptop computer, or telephone device in addition to taking the form of a mobile or wearable device.

Where a computer or data processing system is described as a virtual machine, a virtual device, or a virtual component, the virtual machine, virtual device, or the virtual component operates in the manner of data processing system 200 using virtualized manifestation of some or all components depicted in data processing system 200. For example, in a virtual machine, virtual device, or virtual component, processing unit 206 is manifested as a virtualized instance of all or some number of hardware processing units 206 available in a host data processing system, main memory 208 is manifested as a virtualized instance of all or some portion of main memory 208 that may be available in the host data processing system, and Hard disk drive (HDD) or solid-state drive (SSD) 226a is manifested as a virtualized instance of all or some portion of Hard disk drive (HDD) or solid-state drive (SSD) 226a that may be available in the host data processing system. The host data processing system in such cases is represented by data processing system 200.

### Panoramic X-ray Imaging

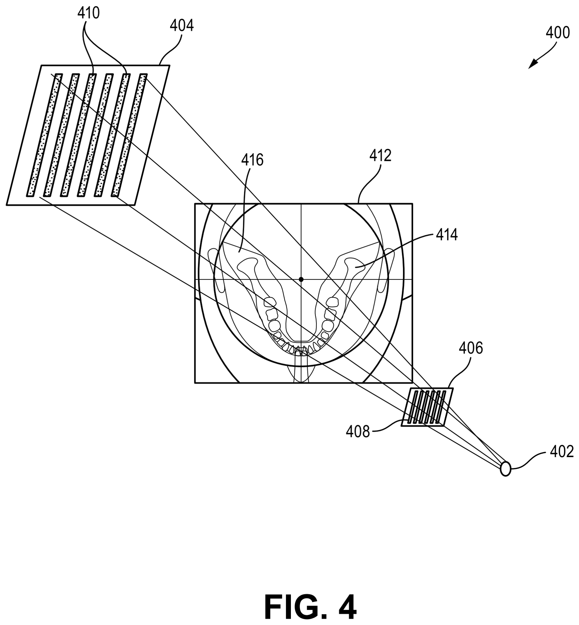

FIG. 4 shows a view of a panoramic X-ray device 400 in accordance with an illustrative embodiment of the present disclosure. The panoramic X-ray device 400 comprises an X-ray source 402 (hereinafter referred as a source 402), a detector or recorder 404, and a collimator 406. The collimator 406 is positioned between the source 402 and the detector 404. The collimator 406 further comprises a plurality of apertures 408 across the periphery of the collimator 406. While imaging, a patient's head 412 is placed between the detector 404 and the collimator 406, and a main X-ray beam is projected from the source 402 onto the collimator 406. The plurality of apertures 408 of the collimator 406 converts the projected main beam into a plurality of individual X-ray projection beams. The collimator 406 forms an individual X-ray projection beam for each respective aperture 408 of the collimator 406.

Further, while imaging, the source 402 with the collimator 406 moves reciprocal to the detector 404 along a predetermined scanning trajectory 416 to image a dental area 414 of the patient's head 412. The scanning trajectory 416 may be determined in advance based on the type of scan and a program of scan such as upper jaw scan, lower jaw scan, complete dental structure scan, etc. The X-ray source 402 is configured to continuously projects the plurality of individual X-ray projection beams onto the dental area 414 through the collimator 406 during their movement along the predetermined scanning trajectory 416. Further, the detector 404 is configured to record a plurality of X-ray projection images 410 corresponding to the plurality of individual X-ray projection beam. Further, the detector 404 is configured to record a plurality of X-ray projection images 410 corresponding to each individual X-ray projection beam along the predetermined scanning trajectory during the scan. For example, each individual X-ray projection beam can generate a response on a corresponding area or region of the detector and the response at the corresponding area or region may be retrieved as a projection image 410, which may be limited to the corresponding area/region or may be a full-sized image that contains information about just the corresponding area or image. Of course, this is not meant to be limiting as other possibilities may be available in light of the descriptions herein.

The panoramic X-ray device 400 reconstructs a panoramic X-ray image of the dental area 414, using one or more of the plurality of X-ray projection images 410 corresponding to one or more different individual X-ray projection beams. The panoramic X-ray device 400 using the processing unit 206 or a graphic processor 210, as shown in FIG. 2, reconstructs the panoramic X-ray image of the dental area 414.

In implementations, the panoramic X-ray device 400 may reconstruct a panoramic X-ray image using one X-ray projection image 410 recorded by one selected individual X-ray projection beam. For example, the panoramic X-ray image may be reconstructed using a first beam, a second beam, or a third beam, etc.

In implementations, the panoramic X-ray device 400 may reconstruct a panoramic X-ray image using a subset of the plurality of X-ray projection images 410 recorded. For example, the panoramic X-ray image may be reconstructed based on a first beam and a third beam.

In implementations, the panoramic X-ray device 400 may reconstruct a panoramic X-ray image based on all of the individual X-ray projection beams. For example, the panoramic X-ray image may be reconstructed using *n* X-ray projection images 410 where *n* is a total number of beams corresponding to the total number of apertures 408 in the collimator 406.

In implementations, the recorded plurality of X-ray projection images on the detector 404 are read-out sequentially or parallel to reconstruct the X-ray projection image of the dental part 414. Since the imaging is a panoramic X-ray imaging which requires a higher read-out rate, the plurality of X-ray projection images on the detector 404 may ideally be read-out in parallel.

FIG. 5A shows a view of a collimator 406 of a panoramic X-ray device 400 in accordance with an illustrative embodiment. In implementations, the collimator 406 may be a plate comprising a plurality of apertures 408 across a body 502 of the plate. The plurality of apertures 408 may be parallel to each other and spread across the body 502 at an equal distance. Further, the collimator 408 may include an X-ray absorbing material coated across the apertures 408 on the body 502. In another embodiment, the collimator 408 may include an X-ray absorbing material coated around the apertures 408 on the body 502.

In implementations, the collimator 406 may be made of an X-ray absorbing material and include a plurality of parallel apertures 408 formed across the body 502 of the collimator 406. The plurality of apertures is spread at an equal distance over the body 502. The absorbing material may include a lead.

It is to be noted that the conventional collimator 306 (undivided aperture), as shown in FIG. 3B includes a single aperture 308 with a wider width. Whereas each of the plurality of apertures 408 of the collimator 406, as shown in FIG. 5A and FIG. 5B in accordance with an illustrative embodiment, may have a width 1/n to the width of the single aperture 308 of the conventional collimator 306. Here, n is the number of apertures 408 of the collimator 406. Thereby, keeping the total width of the plurality of apertures 408 of the illustrative embodiment identical as the width of the single aperture 308 of the conventional collimator 306.Accordingly, the amount of radiation projected through the collimator 406 is kept the same as the amount of radiation projected through the conventional collimator 306.

However, the plurality of apertures 408 spread across the collimator 406 in illustrative embodiment enables a higher angular coverage for each individual dental structure (i.e., teeth) during imaging. Due to the higher angular coverage, more individual structures may be captured orthoradially during the imaging. Additionally, higher angular coverage captures additional reconstruction data for each individual dental structure providing options for reconstruction of the X-ray image with significantly reduced overlapping of dental structures. This results in improvement in a diagnostic option.

Further, the higher angular coverage results in thinner layers in the standard reconstruction, which in turn improves the autofocus options, especially in a molar region of the dental part 414 and increases the overall image quality of the panoramic X-ray images.

FIG. 5B shows a view of a collimator 406 of a panoramic X-ray device 400 in accordance with another illustrative embodiment. Referring to FIG. 5B, the collimator 406 may include a plurality of apertures 408 that are different in size. For instance, one of the plurality of apertures 408 may be different in length than others. Further, the maximum length may be identical to a length of the apertures 408, as shown in FIG. 5A. This configuration decreases a total size of opening within the collimator 406 resulting in decrease in the amount of X-ray radiation exposure to the patient compared to the conventional collimator 306, as shown in FIG. 3B.

Therefore, in present implementations, the collimator 406 with the plurality of apertures 408 increases the angle of coverage during recording while keeping the amount of radiation exposure same or less than the conventional collimator 306, as shown in FIG. 3B.

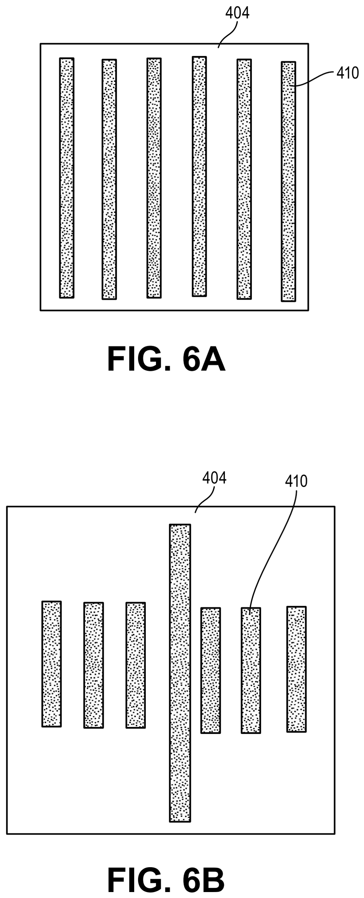

FIG. 6A and FIG. 6B shows a view of a detector 404 of a panoramic X-ray device 400 corresponding to the collimator 406 as shown respectively in FIG. 5A and FIG. 5B.

In implementations, the detector 404 may be a flat plate recorder with a plurality of partial recording regions to record plurality of X-ray projection images 410 corresponding to the plurality of individual X-ray projection beams.

Further, the read-out rate of the plurality of partial recording regions is selected similar to the conventional panoramic imaging or as high as possible.

Further, the panoramic X-ray image is reconstructed using the plurality of X-ray projection images 410 corresponding to each individual X-ray projection beam or the plurality of X-ray projection images 410 corresponding to the combination of two or more different individual X-ray projection beams.

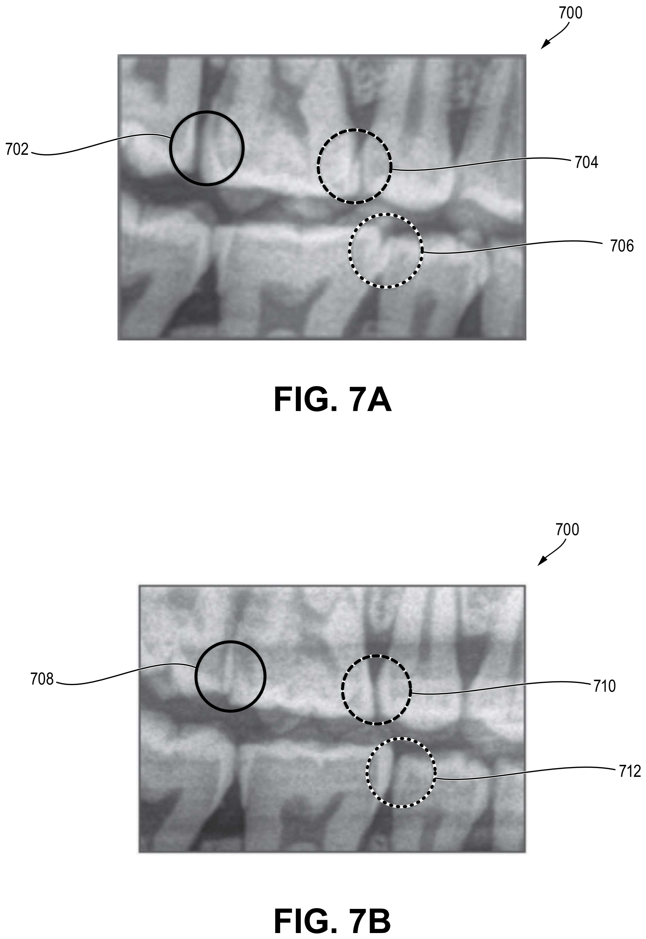

FIG. 7A shows one illustrative image of the X-ray image 700 reconstructed using the X-ray projection images corresponding to one selected individual X-ray projection beam from the plurality of individual X-ray projection beams. FIG. 7B shows another illustrative image of the X-ray image 700 reconstructed using the X-ray projection images corresponding to another selected individual X-ray projection beam of the plurality of individual X-ray projection beams. As shown in FIG. 7A, at a region 702, the individual dental structures are visibly separate, whereas at a region 704 and a region 706, the individual dental structures overlap in the reconstructed X-ray image 700. As shown in FIG. 7B, in region 708, the individual dental structures overlap, whereas at a region 710 and a region 712, the individual dental structures are visibly separate in the reconstructed X-ray image 700. The same regions of the dental part 414 are imaged with different clarity in the reconstructed X-ray image 700 in both FIG. 7A and FIG. 7B due to wide angular coverage provided by the availability of the plurality of individual X-ray projection beams.

Therefore, reconstructing the X-ray image using the X-ray projection images corresponding to different individual X-ray projection beams eliminates or reduces the overlapping issues encountered in conventional imaging methods.

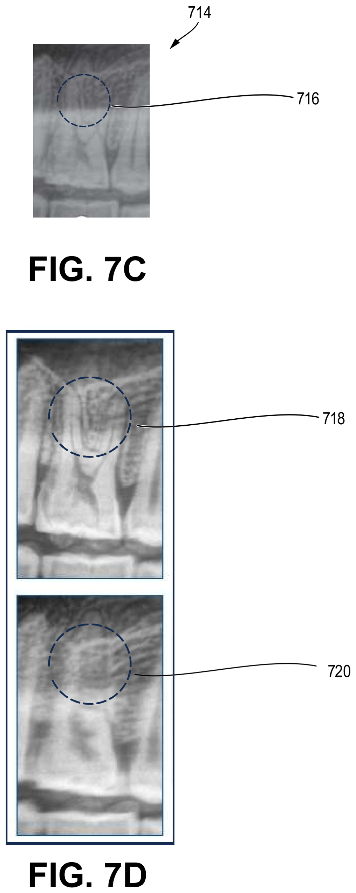

FIG. 7C and FIG. 7D illustrate the focal thickness effect depending on the number of individual X-ray projection beams for reconstruction. The lower the number of multiple X-ray projection beams used, the higher the focal layer thickness. The higher the number of multiple X-ray projection beams used, the lower the focal layer thickness. Based on a small focal layer thickness and adjusted focal layer positions it becomes possible to illustrate the tooth roots independently from each other (see FIG. 7D). The focal layer may be a specific curved, three-dimensional zone (such as one that passes through upper or lower jaw along midpoints of the teeth) where structures are sharply defined on the final image. The focal layer defines the position of the objects along the jawbone to be displayed in the panoramic scan whereas the focal layer thickness defines the amount of blurring of the objects which are not at the focal layer.

FIG. 7C shows an X-ray image 714 reconstructed (based on a default focal layer position) using the one X-ray projection image corresponding to a main/middle X-ray projection beam from the plurality of individual X-ray projection beams. FIG. 7D shows two illustrative images of the X-ray image 714 reconstructed (based on alternative focal layer positions) using the X-ray projection images corresponding to more than one selected individual X-ray projection beam of the plurality of individual X-ray projection beams. As shown in FIG. 7C, at a region 716 (showing the tooth roots), the individual root canals of dental structures are not visibly separate, whereas at a region 718 (tooth roots in the upper image of FIG. 7D) and at a region 720 (tooth roots in the bottom image of FIG. 7D) the individual root canals of dental structures are visibly separate in the reconstructed X-ray image. The same regions of the dental part 414 are imaged with different clarity in the reconstructed X-ray image in both FIG. 7C and FIG. 7D due to wide angular coverage provided by the availability of the plurality of individual X-ray projection beams.

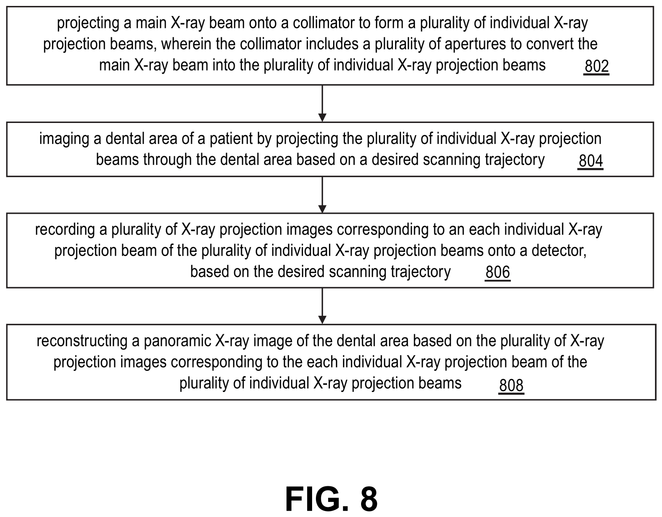

FIG. 8 illustrates a method 800 for panoramic X-ray imaging. In block 802, a main X-ray beam is projected from an X-ray source 402 onto a collimator 406 to form a plurality of individual X-ray projection beams. The collimator 406 includes a plurality of apertures 408 to convert the main X-ray beam into the plurality of individual X-ray projection beams. In block 804, a dental area 414 of a patient is imaged by projecting the plurality of individual X-ray projection beams through the dental area 414 based on a desired scanning trajectory 416. In block 806, a plurality of X-ray projection images 410 corresponding to individual X-ray projection beams of the plurality of individual X-ray projection beams are recorded onto a detector 404. Further, in block 808, a panoramic X-ray image of the dental area 414 is reconstructed based on the plurality of X-ray projection images 410 corresponding to the plurality of individual X-ray projection beams.

### Conclusion

Any specific manifestations of these and other similar example processes are not intended to be limiting to the disclosure. Any suitable manifestation of these and other similar example processes can be selected within the scope of the illustrative embodiments.

Thus, a computer-implemented method, system or apparatus, and computer program product are provided in the illustrative embodiments for panoramic X-ray imaging and other related features, functions, or operations. Where an embodiment or a portion thereof is described with respect to a type of device, the computer-implemented method, system or apparatus, the computer program product, or a portion thereof, are adapted or configured for use with a suitable and comparable manifestation of that type of device.

Where an embodiment is described as implemented in an application, the delivery of the application in a Software as a Service (SaaS) model is contemplated within the scope of the illustrative embodiments. In a SaaS model, the capability of the application implementing an embodiment is provided to a user by executing the application in a cloud infrastructure. The user can access the application using a variety of client devices through a thin client interface such as a web browser, or other light-weight client-applications. The user does not manage or control the underlying cloud infrastructure including the network, servers, operating systems, or the storage of the cloud infrastructure. In some cases, the user may not even manage or control the capabilities of the SaaS application. In some other cases, the SaaS implementation of the application may permit a possible exception of limited user-specific application configuration settings.

The present disclosure may be a system, a method, and/or a computer program product at any possible technical detail level of integration. The computer program product may include a computer-readable storage medium (or media) having computer-readable program instructions thereon for causing a processor to carry out aspects of the present disclosure.

The computer-readable storage medium can be a tangible device that can retain and store instructions for use by an instruction execution device. The computer-readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer-readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer-readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer-readable program instructions described herein can be downloaded to respective computing/processing devices from a computer-readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer-readable program instructions from the network and forwards the computer-readable program instructions for storage in a computer-readable storage medium within the respective computing/processing device.

Computer-readable program instructions for carrying out operations of the present disclosure may be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, including an object oriented programming language such as Smalltalk, C++, or the like, and procedural programming languages, such as the "C" programming language or similar programming languages. The computer-readable program instructions may execute entirely on a dedicated system or user's computer, partly on the user's computer or dedicated system as a stand-alone software package, partly on the user's computer and partly on a remote computer or entirely on the remote computer or server, etc. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer-readable program instructions by utilizing state information of the computer-readable program instructions to personalize the electronic circuitry, in order to perform aspects of the present disclosure.

Aspects of the present disclosure are described herein with reference to flowchart illustrations and/or block diagrams of methods, apparatus (systems), and computer program products according to embodiments of the disclosure. It will be understood that each block of the flowchart illustrations and/or block diagrams, and combinations of blocks in the flowchart illustrations and/or block diagrams, can be implemented by computer-readable program instructions.

These computer-readable program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer-readable program instructions may also be stored in a computer-readable storage medium that can direct a computer, a programmable data processing apparatus, and/or other devices to function in a particular manner, such that the computer-readable storage medium having instructions stored therein comprises an article of manufacture including instructions which implement aspects of the function/act specified in the flowchart and/or block diagram block or blocks.

The computer-readable program instructions may also be loaded onto a computer, other programmable data processing apparatus, or other device to cause a series of operational steps to be performed on the computer, other programmable apparatus or other device to produce a computer-implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present disclosure. In this regard, each block in the flowchart or block diagrams may represent a module, segment, or portion of instructions, which comprises one or more executable instructions for implementing the specified logical function(s). In some alternative implementations, the functions noted in the blocks may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

All features disclosed in the specification, including the claims, abstract, and drawings, and all the steps in any method or process disclosed, may be combined in any combination, except combinations where at least some of such features and/or steps are mutually exclusive. Each feature disclosed in the specification, including the claims, abstract, and drawings, can be replaced by alternative features serving the same, equivalent, or similar purpose, unless expressly stated otherwise.

## Claims

1. A method, comprising:
projecting a main X-ray beam onto a collimator to form a plurality of individual X-ray projection beams, wherein the collimator includes a plurality of apertures that convert the main X-ray beam into the plurality of individual X-ray projection beams;
imaging a dental area of a patient based on the plurality of individual X-ray projection beams using a desired scanning trajectory;
recording a plurality of X-ray projection images corresponding to individual X-ray projection beams of the plurality of individual X-ray projection beams using a detector; and
reconstructing a panoramic X-ray image of the dental area based on one or more X-ray projection images of the plurality of X-ray projection images corresponding to the plurality of individual X-ray projection beams.

2. The method of claim 1, wherein the panoramic X-ray image is reconstructed based on at least one X-ray projection image corresponding to an individual X-ray projection beam of the plurality of individual X-ray projection beams, a combination of the plurality of X-ray projection images corresponding to two or more individual X-ray projection beams of the plurality of individual X-ray projection beams, or a combination of the plurality of X-ray projection images corresponding to all individual X-ray projection beams of the plurality of individual X-ray projection beams.

3. The method of claim 1, wherein the panoramic X-ray image of the dental area is reconstructed based on at least a sequential or a parallel read-out of the plurality of X-ray projection images corresponding to the plurality of individual X-ray projection beams.

4. The method of any one of claims 1-3, wherein an individual X-ray projection beam of each aperture of the plurality of apertures generate an X-ray projection image, a number of the plurality of X-ray projection images combining to form the plurality of X-ray projection images recorded on the detector.

5. The method of any one of claims 1-4, wherein projecting the plurality of individual X-ray projection beams from the plurality of apertures of the collimator to span a predetermined angular range of the dental area.

6. The method of any one of claims 1-5, wherein the collimator further comprises an absorption material disposed across areas of the collimator not occupied by the plurality of apertures.

7. The method of any one of claims 1-6, wherein each aperture of the plurality of apertures of the collimator is of a same size.

8. The method of any one of claims 1-6, wherein each aperture of the plurality of apertures of the collimator is of a different size.

9. The method of any one of claims 1-8, wherein each aperture of the plurality of apertures is equidistant from and parallel to an adjacent aperture.

10. The method of any one of claims 1-9, wherein prior to projecting the main X-ray beam onto the collimator to form a plurality of individual X-ray projection beams, a source beam is projected onto another collimator to generate the main X-ray beam.

11. The method of any one of claims 1-10, wherein imaging a dental area of a patient based on the plurality of individual X-ray projection beams is performed based on any kind of panoramic scan.

12. The method of any one of claims 1-11, wherein sizes of apertures of the plurality of apertures are configured to add up to the size of a single undivided conventional aperture such that a total dose of x-rays received from the plurality of apertures is a same as or less that the total dose received from the single undivided conventional aperture, and such that an angular range covered by the plurality of apertures more than the angular range covered by the single undivided conventional aperture.

13. The method of any one of claims 1-12, wherein the collimator is a combination of a single undivided aperture collimator and a multi-aperture collimator, and a size of an aperture of the single undivided aperture collimator is adjustable.

14. An X-ray device, comprising:
a detector;
a processor; and
a memory, in communication with the processor, with one or more computer program instructions stored on the memory, the computer program instructions, when executed by the processor, cause the x-ray device to perform operations of any one of claims 1-13.

15. A non-transitory computer-readable storage medium storing computer-readable instruction that when executed by a processor of an x-ray device, causes the x-ray device to perform the method of any one of claims 1-13.
